# EUROPEAN PATENT SPECIFICATION

(11) **EP 4 329 685 B1**
(45) Date of publication and mention of the grant of the patent: **03.06.2026**
(21) Application number: 22724987.7
(22) Date of filing: 28.04.2022
(51) Int. Cl.: A61F 5/451, A61F 5/44

(54) **DRAINAGE BAGS WITH AT LEAST ONE FLUID DETECTOR**
DRAINAGEBEUTEL MIT MINDESTENS EINEM FLUIDDETEKTOR
POCHES DE DRAINAGE AVEC AU MOINS UN DÉTECTEUR DE FLUIDE

(30) Priority: 29.04.2021 US 202163181695 P
(43) Date of publication of application: 06.03.2024
(73) Proprietor: Purewick Corporation, El Cajon, California 92020 (US)
(72) Inventor: SHERMER, Charles, Raleigh, North Carolina 27615-4715 (US); NGUYEN, Hoang D., Smyrna, Georgia 30080 (US); VAN ORDEN, Katherine, Decatur, Georgia 30030 (US)
(74) Representative: Hoffmann Eitle
(86) International application number: PCT/US2022/026667
(87) International publication number: WO 2022/232354

(56) References cited:
- US-A1- 2012 041 400
- US-A1- 2020 276 046

## Description

### CROSS-REFERENCE TO RELATED APPLICATION(S)

This application claims priority to U.S. Patent Provisional Application No. 63/181,695 filed on April 29, 2021.

### BACKGROUND

A patient may have limited or impaired mobility such that typical urination processes are challenging or impossible. For example, the patient may have surgery or a disability that impairs mobility. Such a patient may use a urinary catheter, such as a Foley catheter, that drains into a drainage bag to address some of these circumstances. However, the visible presence of urine or other bodily fluids in the drainage bag may cause the patient discomfort and/or embarrassment. US 2012/0041400 A1 discloses a bodily fluid drainage assembly having a fluid bag and one or more covers. The assembly includes one or more volume indicators that defines an opening through which a transparent or semi-transparent portion of the bag can be viewed.

### SUMMARY

Embodiments are directed to drainage bags that include at least one fluid detector, fluid collection systems that include the same, and methods of using the same. According to a first aspect, a drainage bag according to claim 1 is disclosed. The drainage bag includes one or more walls. At least a portion of the one or more walls is opaque. The one or more walls define at least an inlet and a chamber. The drainage bag also includes at least one fluid detector configured to detect at least one of a presence or quantity of one or more bodily fluids in the chamber.

According to second and third aspects, fluid collection systems according to claims 11 and 14 are disclosed. The fluid collection system according to claim 11 includes a wheelchair including a plurality of wheels, a seat, and a back rest, the wheelchair forming a front side and a back side opposite the front side. The fluid collection system also includes a drainage bag. The drainage bag includes one or more walls. At least a portion of the one or more walls is opaque. The one or more walls define at least an inlet and a chamber. The drainage bag also includes at least one fluid detector configured to detect at least one of a presence or quantity of one or more bodily fluids in the chamber.

According to a fourth aspect, a method of using a drainage bag according to claim 15 is disclosed. The method includes receiving a quantity of one or more bodily fluids into a chamber of the drainage bag. The drainage bag includes one or more walls that defines the chamber. At least a portion of the one or more walls is opaque. The method also includes, responsive to receiving the quantity of the bodily fluids into the chamber, indicating at least one of a presence or quantity of the one or more bodily fluids in the chamber with at least one fluid detector.

Features and advantages of the present disclosure will become apparent to those of ordinary skill in the art through consideration of the following detailed description and the accompanying drawings.

### BRIEF DESCRIPTION OF THE DRAWINGS

The drawings illustrate several embodiments of the present disclosure, wherein identical reference numerals refer to identical or similar elements or features in different views or embodiments shown in the drawings.
**FIG. 1A** is a top plan view of a portion of a bodily fluids collection system that includes a drainage bag having at least one fluid detector, according to an embodiment.
**FIGS. 1B** and **1C** are cross-sectional schematics of the drainage bag taken along planes 1B-1B and 1C-1C, respectively, as shown in **FIG. 1A****,** according to an embodiment.
**FIGS. 2-5** are cross-sectional schematics of different drainage bags, according to different embodiments.
**FIG. 6A** is an isometric view of a fluid collection system, according to an embodiment.
**FIG. 6B** is a back plan view of the fluid collection system, according to an embodiment.
**FIG. 7A** is an isometric view of a fluid collection system, according to an embodiment.
**FIG. 7B** is a cross-sectional schematic of the fluid collection system taken along plane 7B-7B as shown in **FIG. 7A****,** according to an embodiment.
**FIG. 8** is a block diagram of a fluid collection system, according to an embodiment.
**FIG. 9A** is a block diagram of a fluid collection system, according to an embodiment.
**FIG. 9B** is a front plan view of the drainage bag, according to an embodiment.
**FIG. 9C** is a cross-sectional schematic of the fluid collection assembly, according to an embodiment.

### DETAILED DESCRIPTION

Embodiments are directed to drainage bags that include at least one fluid detector, fluid collection systems that include the same, and methods of using the same. An example drainage bag includes at least one wall that defines at least a chamber that is configured to receive one or more bodily fluids (*e.g.,* urine, blood, etc.) and an inlet that allows the bodily fluids to enter the chamber. At least a portion of the walls of the drainage bag are opaque thereby reducing a patient's (*i.e.,* a person using the drainage bag) discomfort and/or embarrassment. For example, the walls prevent individuals (*e.g.*, the patient, medical practitioners, visitors, bystanders, etc.) from viewing the bodily fluids in the chamber which would otherwise cause patient discomfort and/or embarrassment. The opaqueness of the walls prevents the patient or medical practitioners from knowing at least one of whether bodily fluids are present in the chamber, the quantity bodily fluids present in the chamber, or when the chamber is full or nearly full. To address this problem, the drainage bag includes at least one fluid detector that is configured to detect at least one of the presence of the bodily fluids in the chamber, the quantity of the bodily fluids that are in the chamber, or when the chamber is full or nearly full. The fluid detector is configured to provide an indication, such as a visual indication, responsive to detecting the bodily fluids.

The drainage bags disclosed herein that include the fluid detector solve several issues of transparent drainage bags. For example, transparent drainage bags allow the bodily fluids present in a chamber thereof to be easily viewed. As such, the presence and quantity of the bodily fluids in the transparent drainage bags may be easily ascertained by the patient and/or other individuals. However, the ability to see the presence and quantity of the bodily fluids in the transparent drainage bags may make the patient uncomfortable and embarrassed. The situation may be exacerbated when the transparent drainage bags are used in public. For instance, transparent drainage bags are not often seen in public and the sight of bodily fluids in the transparent drainage bags may cause stares, pointing, or ridicule. As such, patients who use the transparent drainage bags in public may take steps to prevent bystanders from viewing the bodily fluids in the transparent drainage bags. For example, patients may disposed the transparent drainage bags in an opaque bag. The opaque bag prevents the patient and/or other individuals from determining the presence and quantity of the bodily fluids in the transparent drainage bag without removing the transparent drainage bag from the opaque bag and exposing the transparent drainage bag to the public. Further, the opaque bag may increase the risk that the transparent drainage bag is punctured or leaks due to items disposed in the bag, zippers, etc.

However, walls of the drainage bags disclosed herein are at least partially formed from an opaque material that prevents or at least inhibits viewing of the bodily fluids in the chamber. The drainage bags also include at least one fluid detector that allows for at least one of the presence or quantity of the bodily fluids in such drainage bags to be easily detected. The fluid detector may include, for example, a hydrochromatic ink that changes colors when exposed to the bodily fluids, a pH indicator that changes colors when exposed to the bodily fluids, a hologram, or electrodes. Thus, such drainage bags may be used without causing patient discomfort or embarrassment while also indicating the presence and/or quantity of the bodily fluids therein.

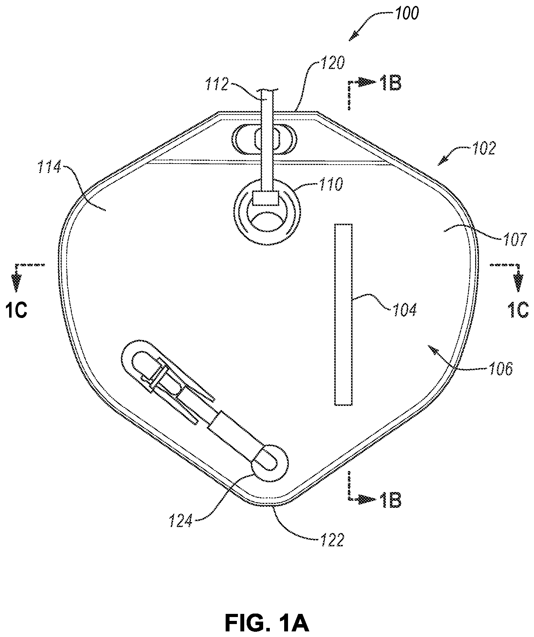

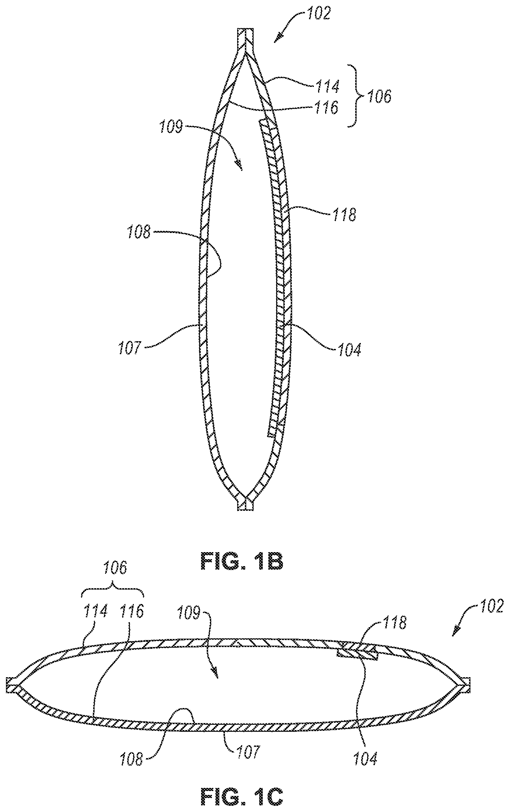

**FIG. 1A** is a top plan view of a portion of a bodily fluids collection system 100 that includes a drainage bag 102 having at least one fluid detector 104, according to an embodiment. **FIGS. 1B** and **1C** are cross-sectional schematics of the drainage bag 102 taken along planes 1B-1B and 1C-1C, respectively, as shown in **FIG. 1A****,** according to an embodiment. The drainage bag 102 includes one or more walls 106 that form a fluid tight container. The walls 106 include at least one exterior surface 107 and at least one interior surface 108 opposite the exterior surface 107. The walls 106 define a chamber 109 (*e.g.,* the interior surface 108 define the chamber 109) and an inlet 110 that allows bodily fluids to enter the chamber 109. At least a portion of the walls 106 are opaque to prevent individuals from seeing the one or more bodily fluids in the chamber 109. The fluid detector 104 is configured to indicate the presence and/or quantity of the bodily fluids in the chamber 109. The bodily fluids collection system 100 includes a drainage tube 112 extending from the inlet 110. The drainage tube 112 allow the drainage bag 102 to be in fluid communication with a fluid collection assembly (not shown) such that the drainage bag 102 receives bodily fluids that were received by the fluid collection assembly. It is noted that the drainage bag 102 may be provided without the drainage tube 112 connected to the inlet 110 or otherwise not have the drainage tube 112 connected to the inlet 110.

In an embodiment, as illustrated, the one or more walls 106 include a front wall 114 and a back wall 116. The plurality of walls 106, may be bonded together along the edges thereof to form a fluid tight container. The plurality of walls 106 may be bonded together using an adhesive, heat sealing, ultrasonic welding, induction sealing, or another suitable attachment.

The walls 106 of the drainage bag 102 may be formed from any suitable fluid impermeable material. In an example, the walls 106 of the drainage bag 102 may be formed from a fluid impermeable polymer (*e.*g., silicone, polyethylene, polypropylene, polytetrafluoroethylene, neoprene, polycarbonate, polyvinyl chloride, etc.), a metal film, natural rubber, another suitable material, or combinations thereof. As such, the walls 106 substantially prevents the bodily fluids from passing through the walls 106. In an example, the walls 106 may be configured to be flexible, rigid, or resilient.

As previously discussed, at least a portion of the walls 106 are opaque. The walls 106 may be opaque when any bodily fluids present in the chamber 109 may not be easily visually identifiable when no backlighting is applied to the drainage bag 102. The opaque portions of the walls 106 may be formed from an opaque material (*e.g*., silicone, metal foil, etc.) or may be treated to make a transparent material opaque (*e.g*., painted or coated with an opaque material). In an embodiment, all of the walls 106 are opaque. In an embodiment, the walls 106 may include at least one opaque portion and at least one transparent portion 118. The transparent portion 118 may be at least partially transparent (*e.g.*, translucent). In an example, the transparent portion 118 may allow the patient or another individual to view the fluid detector 104 when the fluid detector 104 is disposed in the chamber 109 or on the interior surface 108. In such an example, the fluid detector 104 may be at least partially opaque and obstruct at least a portion of the transparent portion 118 such that the fluid detector 104 may inhibit viewing the bodily fluids in the chamber 109. In an example, one side of the drainage bag 102 (*e.g.,* the front wall 114) may be opaque and the other side of the drainage bag 102 (*e.g.,* the back wall 116) may be transparent. During use, the transparent side of the drainage bag 102 may be positioned to be adjacent to a structure (*e.g*., a bed or the portion of a wheelchair) such that the structure inhibits viewing the bodily fluids in the chamber 109 through the back wall 116. However, lifting the drainage bag 102 or otherwise moving the drainage bag 102 away from the structure allows the bodily fluids to be viewed through the transparent side of the drainage bag 102.

Generally, the drainage bag 102 may have any suitable geometry. In the illustrated embodiment, the drainage bag 102 has a generally tear-shaped geometry. However, the drainage bag 102 may have a generally circular geometry (*e.g.*, generally spherical or cylindrical shape when the chamber 109 is filled with bodily fluids), a generally rectangular geometry (*e.g.*, a box-like shape when filled with bodily fluids), or any other suitable shape.

The drainage bag 102 may include a top side 120 and a bottom side 122 opposite the top side 120. During use, the bottom side 122 may be closer to a ground surface (*e.g.,* floor) than the top side 120. The inlet 110 may be located at or near the top side 120 of the drainage bag 102 thereby increasing the quantity of bodily fluids that may be held in the chamber 109 before the level of bodily fluids in the chamber 109 reaches the inlet 110 (*i.e.,* the drainage bag 102 is full). In an embodiment, the drainage bag 102 may include an outlet 124 at or near a bottom side 122 of the drainage bag 102. For example, the outlet 124 may be configured to allow the bodily fluids collected in the drainage bag 102 to flow or drain from the drainage bag 102 (*e.g.,* for collecting or extracting the fluid from the drainage bag 102). For example, the outlet 124 may include the Safety-Flow^{™} outlet device or another similar outlet device.

As previously discussed, the drainage bag 102 includes at least one fluid detector 104. The fluid detector 104 is configured to detect and provide an indication of the presence and/or quantity of the bodily fluids contained in the chamber 109. In an embodiment, the fluid detector 104 is configured to provide a visual indication of the presence and/or quantity of the bodily fluids in the chamber 109. Examples of fluid detectors that provide a visual indication of the presence and/or quantity of the bodily fluids in the chamber 109 include one or more hydrochromatic inks, one or more pH indicators, or holograms. In an embodiment, the fluid detector 104 is configured to provide one or more electronic signals to a device (*e.g.,* a display, cellphone, etc.) that can display that the fluid detector 104 detected.

Referring to **FIGS. 1B** and **1C****,** the fluid detector 104 is disposed in the chamber 109. For example, the fluid detector 104 may be disposed on (*e.g.,* attached) to a portion of the interior surface 108 of the walls 106. Disposing the fluid detector 104 in the chamber 109 allows the fluid detector 104 to directly detect the presence of the bodily fluids in the chamber 109. As such, the fluid detector 104 may include any of the fluid detectors (*e.g.*, hydrochromatic ink or pH indicators) disclosed herein that required direct contact with the bodily fluids to provide the indication. However, it is noted that the fluid detector 104 disposed in the chamber 109 may include a fluid detector that does not require direct contact with the bodily fluids to provide the indication, such as a hologram or electrodes.

In an embodiment, the fluid detector 104 includes one or more hydrochromatic inks. The hydrochromatic ink may be configured to exhibit a color change when the hydrochromatic ink comes in contact with the bodily fluids. The color change in the hydrochromatic ink may be caused by water, a major component of bodily fluids, or any other component of the bodily fluids (*e.g.,* salts, urea, proteins, hormones, metabolites, ammonium, phosphorus, potassium, sulfate, plasma, red blood cells, white blood cells, platelets, etc.). The color change of the hydrochromatic ink may include the hydrochromatic ink exhibiting a first color before the hydrochromatic ink comes in contact with the bodily fluids and a second color after the hydrochromatic ink comes in contact with the bodily fluids. The first color is different than the second color. The first and second colors may be independently selected from transparent, any translucent color (*e.g.,* frosted), or any opaque color. In an example, at least the second color is selected to be an opaque color to prevent bystanders from viewing the bodily fluids in the chamber 109. In an example, the color change of the hydrochromatic ink may be irreversible. That is, after exposing the hydrochromatic ink to the bodily fluids, the hydrochromatic may maintain the second color even after the hydrochromatic ink is no longer in contact with the bodily fluids and is dried. In an example, the color change of the hydrochromatic ink may be reversible such that the hydrochromatic ink may revert back to the first color after the hydrochromatic ink is no longer in contact with the bodily fluids and is dried. The reversible hydrochromatic ink may allow the drainage bag 102 to be reusable (*i.e.,* the bodily fluids may be removed from the chamber 109 thereby allowing more bodily fluids to enter the chamber 109). The first and second color of the hydrochromatic ink and whether the hydrochromatic ink is irreversible or reversible may depend on the chemical composition of the hydrochromatic ink.

In an embodiment, the fluid detector 104 includes a pH indicator. Generally, urine exhibits a pH of about 4.5 to about 8, more particularly about 5 to 7, and more particularly about 6. As such, the pH indicator may be selected to exhibit a color change when the pH is between these pH ranges. As such, the pH indicator may include at least one of Congo red, bromocresol green, methyl purple, bromothymol blue, methyl red, bromocresol purple, bromothymol blue, phenol red, neutral red, azolitmin, phenolphthalein, napththolphthalein, cresol red, any other suitable pH indicator, or combinations thereof.

The pH indicator may be used to diagnosis health conditions that may cause the pH of the bodily fluids to be higher or lower than normal. In an example, the pH indicator may be configured to detect a pH of the bodily fluids that is about 5 or less or about 4.5 or less which may indicate acidosis (*e.g*., diabetic ketoacidosis), dehydration, diarrhea, starvation, or kidney stones. In an example, the pH indicator may be configured to detect a pH of the bodily fluids that is about 7.5 or greater or about 8 or greater which may indicate a urinary tract infection, kidney failure, kidney tubular acidosis, pyloric obstruction, or respiratory alkalosis. In an embodiment, the fluid detector 104 includes a first fluid detector that is configured to detect the presence and/or quantity of the bodily fluids and a second fluid detector that is pH indicator configured to diagnosis health conditions and another fluid detector.

The hydrochromatic inks and/or the pH indicators disclosed herein may be disposed (*e.g.*, printed) on a substrate. In an embodiment, at least a portion of the walls 106 form the substrate. In an embodiment, the substrate may be distinct from the walls 106 of the drainage bag 102. In such an embodiment, substrate may be attached to the walls 106. The substrate that is distinct from the walls 106 may be transparent or opaque. When the substrate is opaque, the substrate may prevent the viewing the bodily fluids in the chamber 109 even when the hydrochromatic ink or the pH indicator is at least partially transparent. In an example, the substrate that is distinct from the walls 106 may exhibit a porosity or textured surface that allows the bodily fluids to reach the hydrochromatic inks or pH indicators. The porosity or textured surface may be necessary when the substrate is opaque and the hydrochromatic inks or pH indicators are positioned between the substrate and the walls 106. The porosity or textured surface may limit the quantity of bodily fluids that contact to hydrochromatic ink or the pH indicator such that the bodily fluids are not easily visually detectable, especially after the hydrochromatic ink or the pH indicator exhibit the color change thereof.

The fluid detector 104 may indicate the quantity of bodily fluids that are present in the drainage bag 102. For example, the fluid detector 104 may include one or more hydrochromatic inks or pH indicators and the fluid detector 104 may extend at least a portion of the distance between the top side 120 and the bottom side 122 of the drainage bag 102. As the drainage bag 102 receives bodily fluids, a portion of the fluid detector 104 may exhibit a color change and the portions of the fluid detector 104 that exhibit the color change may indicate the levels of bodily fluids in the chamber 109. For instance, the chamber 109 may receive a first quantity of bodily fluids in the chamber 109. The first quantity of bodily fluids may be sufficient that the bodily fluids contact a portion of the fluid detector 104. The portions of the fluid detector 104 that come in contact with the first quantity of bodily fluids exhibits a color change while the portions of the fluid detector 104 that do not come in contact with the first quantity of bodily fluids do not exhibit a color change. Thus, the portions of the fluid detector that come in contact with the first quantity of bodily fluids indicates the quantity of bodily fluids in the chamber 109. After receiving the first quantity of bodily fluids, the chamber 109 may receive a second quantity of bodily fluids. The second quantity of bodily fluids causes additional portions of the fluid detector 104 to come in contact with the bodily fluids. Thus, the second quantity of bodily fluids causes further portions of the fluid detector 104 to exhibit a color change. Graduated markings (not shown) may be formed on the exterior surface 107 of the walls 106 to indicate the quantity of bodily fluids in the chamber 109 based on the portions of the fluid detector 104 that exhibits the color change.

As previously discussed, the walls 106 of the drainage bag 102 may include a transparent portion 118 that allows the fluid detector 104 to be seen when the fluid detector 104 is in the chamber 109. The fluid detector 104 may be positioned adjacent to the transparent portion 118. For example, at least a portion of the fluid detector 104 may be attached to the transparent portion 118. The fluid detector 104 may be configured to prevent individuals from viewing the bodily fluids in the chamber 109. In an example, as previously discussed, the fluid detector 104 may be opaque at least after the fluid detector 104 comes in contact with the bodily fluids thereby preventing individuals from viewing the bodily fluids in the chamber 109. In an example, the fluid detector 104 may be configured to cover all or substantially all of the transparent portion 118 thereby restricting individuals from viewing the chamber 109.

The drainage bag 102 may be formed using any suitable technique. In an embodiment, the transparent portion 118 may be formed by forming a hole in opaque walls and attaching a transparent fluid impermeable material to the opaque walls. In an embodiment, the transparent portion 118 may be formed by treating (*e.g.*, painting or coating) all of one or more transparent walls except for a portion of the transparent walls that form the transparent portion 118. In an embodiment, the fluid detector 104 may be disposed (*e.g.*, adhesively attached, printed, etc.) on the interior surface 108 of the walls 106. After disposing the fluid detector 104 on the interior surface 108, one or more edges of the walls 106 may be bonded together.

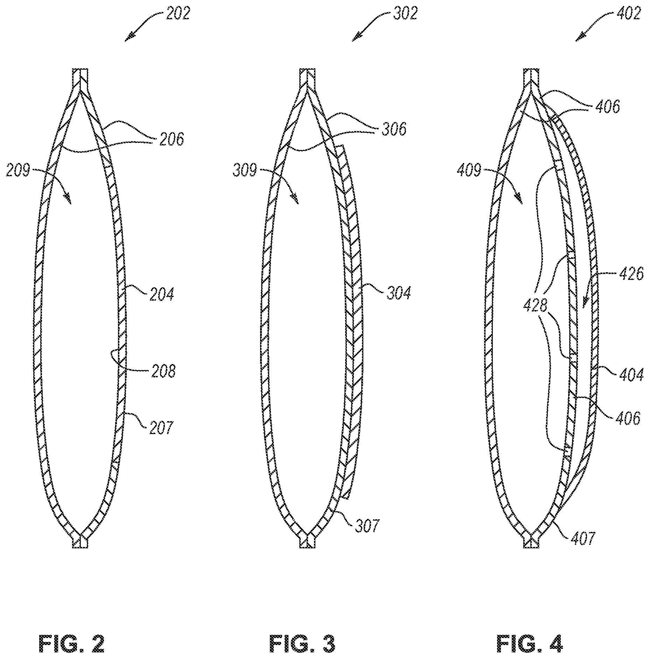

The drainage bags disclosed herein may exhibit structures other than the structures illustrated in **FIGS. 1B** and **1C****.** For example, **FIGS. 2-5** are cross-sectional schematics of different drainage bags, according to different embodiments. Except as otherwise disclosed herein, the drainage bags illustrated in **FIGS. 2-5** are the same or substantially similar to any of the drainage bags disclosed herein. For example, the drainage bags illustrated in **FIGS. 2-5** may include at least one fluid detector and one or more walls defining a chamber and an inlet.

Referring to **FIG. 2****,** the drainage bag 202 includes at least one fluid detector 204 that forms at least a portion of the one or more walls 206. In other words, the at least one fluid detector 204 forms at least a portion of the at least one exterior surface 207 and the at least one interior surface 208 that defines the chamber 209. As such, the fluid detector 204 directly contacts the bodily fluids. The walls 206 do not need to include a transparent portion since the fluid detector 204 forms part of the exterior surface 207 of the walls 206.

The drainage bag 202 may be formed by forming a hole in the walls 206 of the drainage bag 202. The hole may be formed by cutting or otherwise removing portions of the walls 206 to form the hole, the walls 206 may be provided with the hole, or the hole may be the space between edges of the walls 206 before the walls 206 are bonded together. After forming the hole, the fluid detector 204 may be disposed in the hole or over the hole (*e*.*g.,* the fluid detector 204 overlaps portions of the walls 206 that define the hole). The fluid detector 204 may then been attached to the portions of the walls 206 that define the hole in a fluid tight manner. For example, the fluid detector 204 may be attached to the walls 206 using tape, an adhesive, ultrasonic welding, or any other suitable attachment technique.

Referring to **FIG. 3****,** the drainage bag 302 include one or more walls 306 and at least one fluid detector 304. The fluid detector 304 is disposed (*e.g.*, attached, printed, etc.) on a portion of an exterior surface 307 of the walls 306. The fluid detector 304 is not in direct contact with any bodily fluids that are present in the chamber 309 since the fluid detector 304 is disposed on the exterior surface 307. As such, the fluid detector 304 is selected to be a fluid detector 304 that does not require direct contact with the bodily fluids to provide the visual indication.

In an embodiment, the fluid detector 304 is a hologram. In such an embodiment, the hologram may provide a first visual indication to an individual when the hologram is in a first position and a second visual indication to the individual when the hologram is in a second position that is different than the first position. The hologram may be in the first position when no bodily fluids are in the chamber 309. Disposing bodily fluids in the chamber 309 may cause the walls 306 to bulge outwardly, thereby changing the hologram from the first position to the second position. As such, the second visual indication of the hologram may indicate the presence of the bodily fluids in the chamber 309. In an example, the hologram may only be in the second position when the chamber 309 is full or substantially full. In an example, the fluid detector 304 may include a plurality of holograms and each hologram may provide the second visual indications thereof when a certain quantity of bodily fluids are present in the chamber 309. For instance, the fluid detector 304 may include a first hologram that provides the second visual indication thereof when a first quantity of bodily fluids are in the chamber 309, a second hologram that provides the second visual indication thereof when a second quantity of bodily fluids are in the chamber 309, and so forth.

In an embodiment, the fluid detector 304 may include a visual indicator disposed on or formed on the walls 306 (*e.g.,* a colored, textured, or otherwise distinguishable portion of the walls 306). The walls 306 may include a fold formed therein that covers the visual indicator when substantially no bodily fluids are disposed in the chamber 309. Disposing bodily fluids in the chamber 309 may cause the fold to unfold thereby exposing the visual indicator. In an example, the fluid detector 304 may include a plurality of folds and each fold may display the visual indicator thereof when a certain quantity of bodily fluids are present in the chamber 309. For instance, the fluid detector 304 may include a first fold that displays the visual indicator thereof when a first quantity of bodily fluids are in the chamber 309, a second fold that displays the visual indicator thereof when a second quantity of bodily fluids are in the chamber 309, and so forth.

Referring to **FIG. 4****,** the drainage bag 402 include one or more walls 406 and at least one fluid detector 404. The fluid detector 404 is disposed (e.g., attached, printed, etc.) on a portion of an exterior surface 407 of the walls 406. The fluid detector 404 may be attached to the one or more walls 406 to form a secondary chamber 426 that is substantially fluid tight. For example, the edges of the fluid detector 404 may be attached to the walls 406 and the fluid detector 404 may be formed from a fluid impermeable material. The portions of the walls 406 adjacent to the secondary chamber 426 may define one or more holes 428 formed therein. The holes 428 allows bodily fluids in the chamber 409 to flow from the chamber 409 to the secondary chamber 426. The bodily fluids that are in the secondary chamber 426 may come in direct contact with the fluid detector 404 and may change the shape of the fluid detector 404. As such, the fluid detector 404 may include any of the fluid detectors disclosed herein.

In an embodiment, the fluid detector 404 may be used to retrofit an opaque drainage bag. For example, the opaque drainage bag may have one or more holes 428 formed thereon. After forming the holes 428, the drainage bag 402 is formed by attaching the fluid detector 404 to a portion of the walls 406 in a fluid tight manner to form the secondary chamber 426. The fluid detector 404 is attached to the walls 406 such that the holes 428 are adjacent to and in fluid communication with the secondary chamber 426.

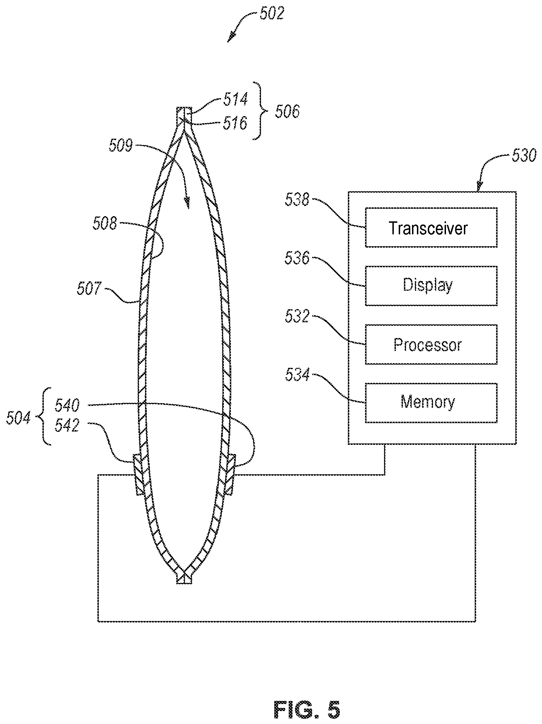

Referring to **FIG. 5**, the drainage bag 502 includes one or more walls 506 defining a chamber 509. At least one fluid detector 504 is attached to the walls 506 (*e.g.,* an exterior and/or interior surface 507, 508) of the walls 506, disposed in the walls 506, forms part of the walls 506 (similar to the fluid detector 304 of **FIG. 3****),** or disposed in the chamber 509. The fluid detector 504 is configured to detect the presence and/or quantity of the bodily fluids and generate one or more electric signals responsive to the detecting. The fluid detector 504 may be coupled (*e.g*., wired or wirelessly) to at least one electronic device 530 and is configured to output the electric signals to the electronic device 530. The electronic device 530 is configured to receive the electronic signals and provide a visual indication responsive to receiving the electric signals. For example, the electronic device 530 may include at least one processor 532, memory 534 (*e.g*., non-transitory memory) storing one or more operational instructions, a display 536, and at least one transceiver 538. Responsive to receiving the electric signals, the processor 532 may execute one or more of the operational instructions which may cause the processor 532 to analyze the electronic signals and/or display the information detected by the fluid detector 504 (*e.g.,* the presence and/or quantity of bodily fluids) on the display 536. The processor 532 may also cause at least a portion of the received electronic signals may be stored in the memory 534. The transceiver 538 may be used to receive the electronic signals from the fluid detector 504 and/or may transmit data (*e.g.,* at least a portion of the electronic signals) to another electronic device.

In an embodiment, the fluid detector 504 includes at least one capacitor. Each capacitor may include a first electrode 540 and a second electrode 542. In an example, the first and second electrodes 540, 542 are disposed on the front and back walls, 514, 516 of the walls 506, respectively. The presence and/or quantity of the bodily fluids may be detected based on measuring at least one property of the capacitor. The property of the capacitor that may be measured includes at least one of charge density, capacitance, leakage current, or any other properties of the capacitor. For example, the first and second electrode 540, 542 may be spaced from each other by a first distance and one or more first materials (*e.g.*, the walls 506 and, optionally, air) before one or more bodily fluids are introduced into the chamber 509. Disposing the bodily fluids into the chamber 509 may cause the distance and/or materials between the first and second electrodes 540, 542 to change which, in turn, may cause the properties of the capacitor that are being measured to change. In an example, the bodily fluids may cause the distance between the first and second electrodes 540, 542 to increase from the first distance to a second distance. The increased distance between the first and second electrodes 540, 542 may change the properties of the capacitor that are being measured. It is noted that the second distance may correspond to the quantity of bodily fluids that are present in the chamber 509 since increasing the quantity of bodily fluids in the chamber 509 may increase the distance between the first and second electrodes 540, 542. In an example, the bodily fluids may be disposed between the first and second electrodes 540, 542 such that the first and second electrodes 540, 542 are separated by a second material (*e.g.*, the walls 506 and the bodily fluids). The second material may exhibit a dielectric constant (*e.g.*, relative permittivity) that is different than the first material which may change the property of the capacitor that is being measured.

The fluid detector 504 may include detectors other than at least one capacitor. For example, the fluid detector 504 may include at least one of an ultrasound transducer that can detect the bodily fluids, an electronic pH sensor configured to detect the pH of the bodily fluids, a photodetector disposed in the chamber 509 or configured to image at least one hydrochromatic inks and/or at least one pH indicator, a chemiresistor, an electronic nose, a holographic sensor, a humistor, a hydrometer, or any other suitable sensor.

In a particular embodiment, the drainage bags disclosed herein may be used with a patient that uses a wheelchair. The patient may use the wheelchair to access public places. Using the wheelchair in public places may cause the drainage bags to be visible to individuals. However, as previously discussed herein, the opaque walls of the drainage bags disclosed herein may prevent individuals from seeing the bodily fluids in the drainage bag when the drainage bag is used with a wheelchair. However, the particular configuration and structure of the drainage bag may depend on the placement of the drainage bag on the wheelchair.

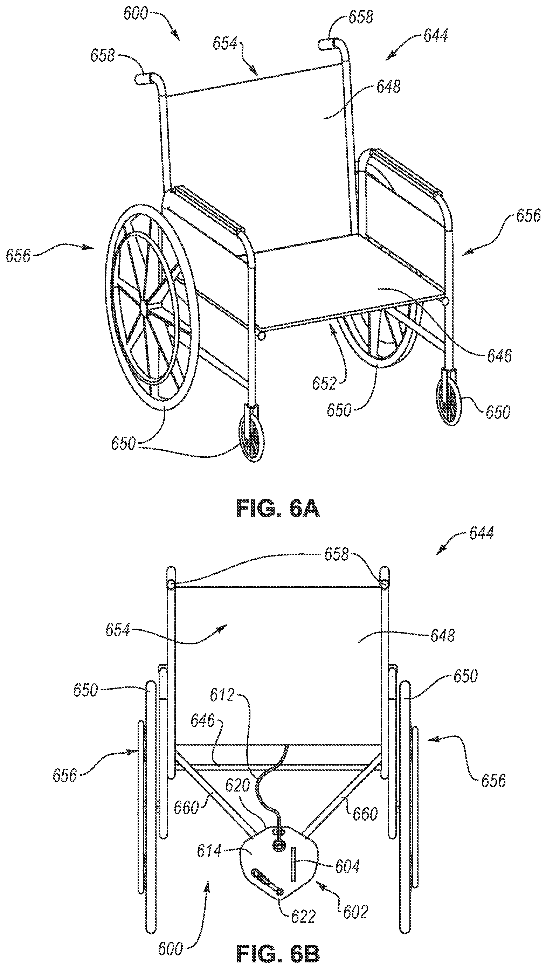

For example, **FIG. 6A** is an isometric view of a fluid collection system 600, according to an embodiment. **FIG. 6B** is a back plan view of the fluid collection system 600, according to an embodiment. The fluid collection system 600 includes a drainage bag 602, a drainage tube 612 in fluid communication with the drainage bag 602, and a wheelchair 644. Except as otherwise disclosed herein, the drainage bag 602 is the same or substantially similar to any of the drainage bags disclosed herein.

The wheelchair 644 may include any generic or specially made wheelchair. For example, the wheelchair 644 may include a manual-self propelled wheelchair, a manual attendant-propelled wheelchair, a powered wheelchair, a mobility scooter, a single-art drive wheelchair, a reclining and/or tilting wheelchair, a standing wheelchair, a sports wheelchair, a wheelchair stretcher, an all-terrain wheelchair, a smart wheelchair, or any other wheelchair. The wheelchair 644 may include a seat 646, a back rest 648, and one or more wheels 650. The wheelchair 644 may include a front side 652, and a back side 654 opposite the front side 652. The front side 652 of the wheelchair 644 is the side of the wheelchair 644 where the legs of the patient are configured to be positioned and may include one or more leg rests (not shown). The back side 654 of the wheelchair 644 is the side of the wheelchair 644 with the back rest 648 and may include one or more handles 658.

In an embodiment, the drainage bag 602 is configured to be positioned on the back side 654 of the wheelchair 644. For example, the drainage bag 602 may be dangled using one or more straps 660 or may be positioned in a pocket. The drainage bag 602 includes a back side (not shown, obscured), a front side 614 opposite the back side 616, a top side 620, and a bottom side 622 opposite the top side 620. The back side 616 of the drainage bag 602 generally faces the front side 652 of the wheelchair 644 and the back side 616 of the drainage bag 602 faces the same direction as the back side 654 of the wheelchair 644. The bottom side 622 of the drainage bag 602 generally faces a ground on which the wheelchair 644 is disposed.

In an embodiment, the fluid detector 604 of the drainage bag 602 may be disposed on at least one of the front side 614 or the top side 620 of the drainage bag 602. Positioning the fluid detector 604 on the front side 614 or the top side 620 allows the fluid detector 604 to be easily viewed by an individual pushing the wheelchair 644 or an individual (*e.g.*, medical practitioner) who stands adjacent to the back side 654 of the wheelchair 644. As such, the fluid detector 604 may be easily and quickly examined. In an embodiment, the fluid detector 604 may be disposed on a side of the drainage bag 602 other than the front side 614 or the top side 620 of the drainage bag 602. Positioning the fluid detector 604 on another side of the drainage bag 602 may require handling (*e.g.*, lifting or twisting) of the drainage bag 602 to view the fluid detector 604 thereby increasing the time and effort required to examine the fluid detector 604. The increased time and effort required to examine the fluid detector 604 may result in the individual checking the fluid detector 604 less often or failing to notice an increase in the rate at which the drainage bag 602 is being filled thereby increasing the risk that the drainage bag 602 becomes overfilled than if the fluid detector 604 is disposed on the front or top side 620, 622 of the drainage bag 602.

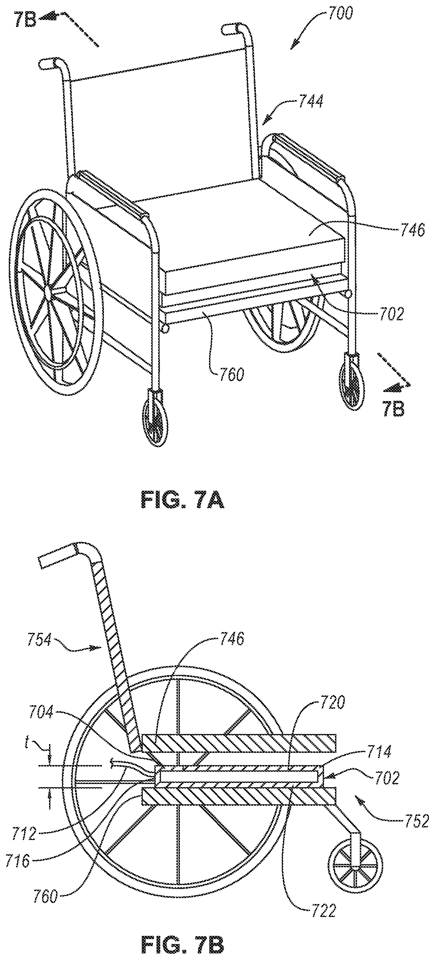

The drainage bags disclosed herein may be disposed in other locations on wheelchairs. For example, **FIG. 7A** is an isometric view of a fluid collection system 700, according to an embodiment. **FIG. 7B** is a cross-sectional schematic of the fluid collection system 700 taken along plane 7B-7B as shown in **FIG. 7A****,** according to an embodiment. Except as otherwise disclosed herein, the fluid collection system 700 may be the same or substantially similar to any of the fluid collection systems disclosed herein. For example, the fluid collection system 700 may include a drainage bag 702, a drainage conduit 712 in fluid communication with the drainage bag 702, and a wheelchair 744. Except as otherwise disclosed herein, the drainage bag 702, the drainage conduit 712, and the wheelchair 744 are the same or substantially similar to any of the drainage bags, drainage conduits, and wheelchairs disclosed herein.

The wheelchair 744 includes a shelf 760 that is configured and/or able to hold the drainage bag 702 thereon. The shelf 760 may be positioned under the seat 746 of the wheelchair 744. The drainage bag 702 may be disposed on the shelf.

In an embodiment, the distance between the shelf 760 and the seat 746 may not be sufficient for the drainage bag 702 to dangle such that the length or width of the drainage bag 702 (*e.g.,* the two largest dimensions of the drainage bag 702 measure perpendicularly to each other) is generally parallel to the direction of gravity, as illustrated in **FIGS. 1A-5** and **6B.** Instead, the thickness t of the drainage bag 702, which is measure perpendicularly and is less than both of the length and the width of the drainage bag 702, may be generally parallel to gravity.

The drainage bag 702 illustrated in **FIGS. 7A** and **7B** includes a front side 714, a back side 716 opposite the front side 716, a top side 720, and a bottom side 722. The front and back sides 714, 716 generally face the same directions and the front and back sides 752, 754 of the wheelchair respectively. The bottom side 722 may contact the shelf 760 (*e.g.,* the bottom side 722 may be transparent since the shelf blocks views of the bottom side 722) and the top side 720 may generally face the seat 746. The fluid detector 704 may be disposed on at least one of the front side 714, the back side 716, or the top side 720. In an example, the shelf 760 may have an opening on the front side 752 of the wheelchair 744. A patient sitting on the seat 746 may easily view the fluid detector 704 by leaning forward when the fluid detector 704 is on the front side 714 and/or the top side 720 proximate the front side 714. Similarly, an individual (*e.g.*, medical practitioner) adjacent to the front side 752 of the wheelchair 744 may easily view the fluid detector 704 when the fluid detector 704 is on the front side 714 and/or the top side 720 proximate the front side 714. In an example, the shelf 760 may have an opening on the back side 754 of the wheelchair 744. A person pushing the wheelchair 744 or an individual standing adjacent to the back side 754 of the wheelchair 744 may easily view the fluid detector 704 if the fluid detector 704 is on the back side 716 and/or the top side 720 proximate the back side 716. In an example, the drainage bag 702 may include at least one first fluid detector disposed on the front side 714 and/or the top side 720 proximate the front side 714 and at least one second fluid detector disposed on the back side 716 and/or the top side 720 proximate the back side 716.

The drainage bags may be used in applications that do not include a wheelchair. In an embodiment, the drainage bags disclosed herein may be used with a patient that is on a bed. In such an embodiment, the drainage bag may be attached to the bed or an object near the bed. For instance, the drainage bag may be dangled from the bed or an object near the bed similar to the drainage bag 602 or may be disposed on a shelf similar to the drainage bag 702. The opaque walls of the drainage bag may prevent visitors to the patient, individuals walking by the patient, or other individuals from viewing the bodily fluids in the chamber of the drainage bag. In an embodiment, the drainage bags disclosed herein may be used with a patient that uses as IV pole. For example, the drainage bag may be attached near the base of the IV pole and the IV pole may allow the patient to walk about a hospital or other facility. The opaque walls may of the drainage bag may prevent visitors to the patient, individuals walking by the patient, individuals the patient walks past in the halls, or other individuals from viewing the bodily fluids in the chamber of the drainage bag. In an embodiment, the drainage bags disclosed herein may be used with patients who are able to walk. In such an embodiment, the drainage bag may be attached to or include a strap that may be positioned over the patient's shoulders, attached to the patient's legs, disposed in a bag, or otherwise carried by the patient. Again, the opaque walls of the drainage bag prevent individuals from viewing the bodily fluids in the drainage bag. In any of the above embodiments, the drainage bag includes at least one fluid detector that allows the patient or another individual to determine the presence and/or quantity of bodily fluids in the chamber of the drainage bag.

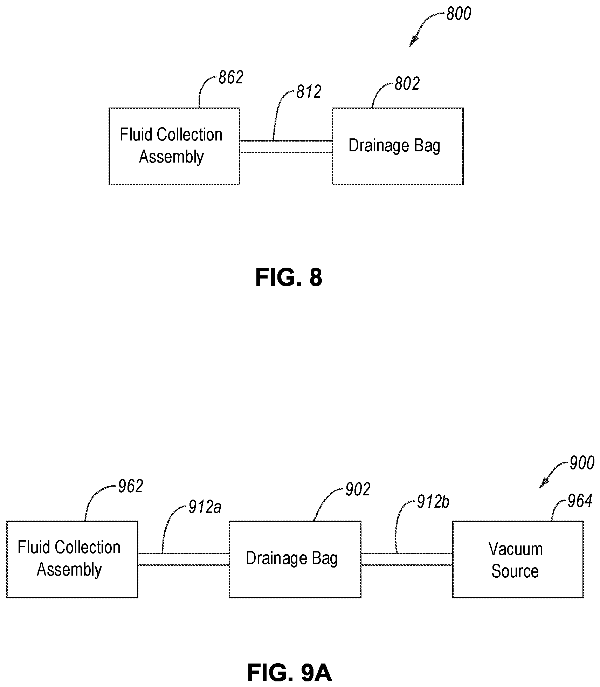

As previously discussed, the drainage bags disclosed herein may be connected to a fluid collection assembly. **FIG. 8** is a block diagram of a fluid collection system 800, according to an embodiment. The fluid collection system 800 includes a drainage bag 802. The drainage bag 802 may be the same or substantially similar to any of the drainage bags 802 disclosed herein. The fluid collection system 800 also includes a fluid collection assembly 862. The fluid collection assembly 862 may include any device that is configured to receive bodily fluids from a patient. Examples of the fluid collection assembly 862 include a Foley catheter, a condom catheter, a PureWick female external catheter available from Becton Dickinson, or any other suitable fluid collection assembly 862. The fluid collection assembly 862 may be in fluid communication with the drainage bag 802 via at least one drainage tube 812 such that at least some of the bodily fluids received by the fluid collection assembly 862 is disposed in the chamber of the drainage bag 802.

In some examples, the drainage bags disclosed herein may form part of a fluid collection system that includes a vacuum source that is configured to pull bodily fluids received by the fluid collection assembly into the drainage bag. **FIG. 9A** is a block diagram of a fluid collection system 900, according to an embodiment. The fluid collection system 900 includes a drainage bag 902, a fluid collection assembly 962, and a vacuum source 964. The drainage bag 902 and the fluid collection assembly 962 may be in fluid communication with each other via at least one first drainage tube 912a and drainage bag 902 and the vacuum source 964 may be in fluid communication with each other via at least one drainage tube 912b. During operation, the fluid collection assembly 962 may receive one or more bodily fluids. The vacuum source 964 may apply a vacuum pressure to the fluid collection assembly 962 (*e.g.,* a chamber of the fluid collection assembly 962) that pulls the bodily fluids from the fluid collection assembly 962, through the first drainage tube 912a, and deposits the bodily fluids in the chamber of the drainage bag 902.

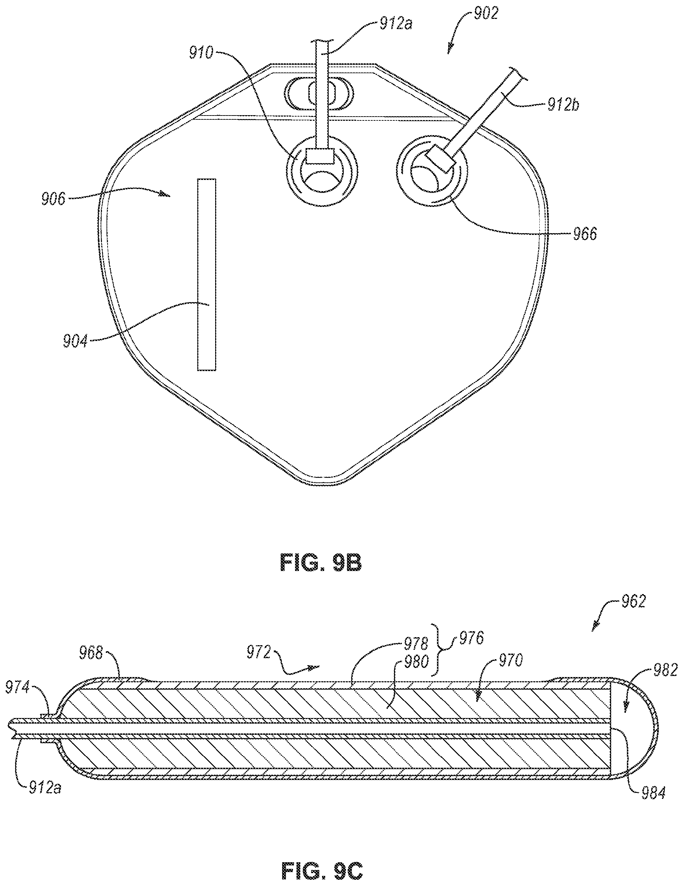

**FIG. 9B** is a front plan view of the drainage bag 902, according to an embodiment. Except as otherwise disclosed herein, the drainage bag 902 is the same or substantially similar to any of the drainage bags disclosed herein. For example, the drainage bag 902 includes at least one fluid detector 904 and one or more walls 906 defining at least a chamber (not shown) and at least one inlet 910. The inlet 910 may be attached to the first drainage tube 912a such that the drainage bag 902 is in fluid communication with the fluid collection assembly 962 shown in **FIG. 9A****.** The one or more walls 906 also define a vacuum port 966. The vacuum port 966 may be attached to the second drainage tube 912b such that the drainage bag 902 is in fluid communication with the vacuum source 964 shown in **FIG. 9A****.** The walls 906 may be rigid or semi-rigid such that the vacuum pressure applied to the chamber from the vacuum source 964 does not cause the drainage bag 902 to collapse which, in turn, would cause the vacuum pressure to remove the bodily fluids from the chamber.

**FIG. 9C** is a cross-sectional schematic of the fluid collection assembly 962, according to an embodiment. The fluid collection assembly 962 include a fluid impermeable barrier 968. The fluid impermeable barrier 968 defines a chamber 970, at least one opening 972, and a fluid outlet 974. The fluid collection assembly 962 also includes at least one porous material 976 disposed in the chamber 970. In the illustrated embodiment, the porous material 976 includes a fluid permeable membrane 978 (*e.g.,* gauze) that extends across the opening 972 and a fluid permeable support 980 (*e.g.,* spun nylon fibers) that support the fluid permeable membrane 978, though it is noted that the porous material 976 may include less than or more than the two layers illustrated in **FIG. 9C****.** The porous material 976 may occupy all of or only a portion of the chamber 970. When the porous material 976 only occupies a portion of the chamber 970, the unoccupied portions of the chamber 970 may form a fluid reservoir 982. The first drainage tube 912a may be positioned in or through the fluid outlet 974. In an embodiment, as illustrated, the porous material 976 may define a bore and the first drainage tube 912a may be positioned through the bore such that an inlet 984 of the first drainage tube 912a is positioned adjacent to the fluid reservoir 982.

During operation, one or more bodily fluids may be discharged from a patient, such as from the urethral opening of the patient. The bodily fluids may flow into the chamber 970 (*e.g.,* through the opening 972) and be received into the porous material 976. The bodily fluids may flow through the porous material 976 towards the inlet 984 of the first drainage tube 912a (*e.g*., towards the reservoir 982). The vacuum pressure may remove the bodily fluids from the chamber 970, through the first drainage tube 912a, and deposit the bodily fluids in the chamber of the drainage bag 902.

It is noted that the fluid collection assembly 962 illustrated in **FIG. 9C** is merely one example of a fluid collection assembly that may be used in the fluid collection system 900 and the other fluid collection systems disclosed herein. Additional examples of fluid collection assemblies that may be used in any of the fluid collection systems disclosed herein are disclosed in U.S. Provisional Patent Application No. 63/067,542 filed on August 19, 2020, U.S. Patent Application No. 16/433,773 filed on June 6, 2019, U.S. Patent Application No. 15/612,325 filed on June 2, 2017; U.S. Patent Application No. 15/260,103 filed on September 8, 2016; U.S. Patent Application No. 16/452,258 filed on August 18, 2016; U.S. Patent No. 10,376,406 filed on August 27, 2016; and U.S. Patent No. 10,390,989 filed on March 19, 2014. For example, a fluid collection assembly may be configured to be used with a penis. In such an example, the fluid collection assembly may be positioned such that the penis is received within the chamber or the opening is positioned adjacent to the penis. The fluid collection assembly may also be connected to any of the drainage bags disclosed herein such that at least some of the bodily fluids received

While various aspects and embodiments have been disclosed herein, other aspects and embodiments are contemplated. The various aspects and embodiments disclosed herein are for purposes of illustration and are not intended to be limiting.

Terms of degree (*e.g.*, "about," "substantially," "generally," etc.) indicate structurally or functionally insignificant variations. In an example, when the term of degree is included with a term indicating quantity, the term of degree is interpreted to mean ± 10%, ±5%, or +2% of the term indicating quantity. In an example, when the term of degree is used to modify a shape, the term of degree indicates that the shape being modified by the term of degree has the appearance of the disclosed shape. For instance, the term of degree may be used to indicate that the shape may have rounded corners instead of sharp corners, curved edges instead of straight edges, one or more protrusions extending therefrom, is oblong, is the same as the disclosed shape, etc.

## Claims

1. A drainage bag (102-902), comprising one or more walls (106-506; 906) including a front wall (114) and a back wall (116), at least a portion of the front wall is opaque, the one or more walls defining at least:
an inlet (110;910); and
a chamber (109-509, 970); and
at least one fluid detector (104-704,904) configured to detect at least one of a presence or quantity of one or more bodily fluids in the chamber,
**characterised in that**
the at least one fluid detector is opaque; and
wherein at least the portion of the front wall that is opaque and the at least one fluid detector prevent viewing of bodily fluids in the chamber.

2. The drainage bag of claim 1, wherein the at least one fluid detector is at least partially disposed on at least one exterior surface (107-407) of the one or more walls.

3. The drainage bag of claim 2, wherein the at least one fluid detector and the one or more walls define a secondary chamber (426), the one or more walls define one or more holes that allow the one or more bodily fluids in the chamber to flow through the one or more walls to the secondary chamber.

4. The drainage bag of any one of claims 1-3, wherein the at least one fluid detector is disposed on at least one interior surface of the one or more walls, and the at least one fluid detector is disposed on a transparent portion of the one or more walls.

5. The drainage bag of any one of claims 1-4, wherein the at least one fluid detector forms part of the one or more walls.

6. The drainage bag of any one of claims 1-5, wherein the at least one fluid detector includes at least one hydrochromatic ink.

7. The drainage bag of any one of claims 1-6, wherein the at least one fluid detector includes at least one pH indicator.

8. The drainage bag of any one of claims 1-7, wherein the at least one fluid detector includes at least one hologram.

9. The drainage bag of any one of claims 1-8, wherein the at least one fluid detector includes a capacitor.

10. The drainage bag of any one of claims 1-9, wherein the at least one fluid detector indicates a quantity of the one or more bodily fluids in the chamber.

11. A fluid collection system (600;700), comprising:
a wheelchair (644;744) including a plurality of wheels (650), a seat (646;746), and a back rest (648), the wheelchair forming a front side (652;752) and a back side (654;754) opposite the front side; and
the drainage bag (102-902) of any one of claims 1-10.

12. The fluid collection system (600;700) of claim 11, further comprising:
a fluid collection assembly; and
at least one drainage tube extending from the inlet of the drainage bag to the fluid collection assembly.

13. The fluid collection system of claim 12, wherein the fluid collection assembly (962) includes:
at least one fluid impermeable barrier (968) at least defining a chamber, at least one opening, and a fluid outlet; and
at least one porous material disposed in the chamber.

14. A fluid collection system (800;900), comprising:
a fluid collection assembly (862;962);
the drainage bag of any one of claims 1-10 (102-902); and
at least one drainage tube (812; 912a) extending from the inlet of the drainage bag to the fluid collection assembly.

15. A method of using a drainage bag (102-902), the method comprising:
receiving a quantity of one or more bodily fluids into a chamber of the drainage bag, the drainage bag including one or more walls (106-506; 906) that defines the chamber (109-509;970), the one or more walls including a front wall (114) and a back wall (116), at least a portion of the front wall (114) is opaque; and
responsive to receiving the quantity of the bodily fluids into the chamber, indicating at least one of a presence or quantity of the one or more bodily fluids in the chamber with at least one fluid detector (104-704;904),
wherein the at least one fluid detector is opaque; and
wherein at least the portion of the front wall that is opaque and the at least one fluid detector prevent viewing of bodily fluids in the chamber.

## Patentansprüche

1. Drainagebeutel (102-902), umfassend
eine oder mehrere Wände (106-506; 906) einschließlich einer Vorderwand (114) und einer Rückwand (116), wobei mindestens ein Abschnitt der Vorderwand undurchsichtig ist, wobei die eine oder die mehreren Wände mindestens Folgendes definieren:
einen Einlass (110;910); und
eine Kammer (109-509, 970); und
mindestens einen Fluiddetektor (104-704, 904), der ausgebildet ist, um mindestens entweder das Vorhandensein oder die Menge einer oder mehrerer Körperfluide in der Kammer zu erfassen,
**dadurch gekennzeichnet, dass**
der mindestens einen Fluiddetektor undurchsichtig ist; und
wobei mindestens der undurchsichtige Abschnitt der Vorderwand und der mindestens eine Fluiddetektor den Blick auf Körperfluide in der Kammer verhindern.

2. Drainagebeutel nach Anspruch 1, wobei der mindestens eine Fluiddetektor mindestens teilweise auf mindestens einer Außenfläche (107-407) der einen oder mehreren Wände angeordnet ist.

3. Drainagebeutel nach Anspruch 2, wobei der mindestens eine Fluiddetektor und die eine oder mehrere Wände eine Sekundärkammer (426) definieren, wobei die eine oder mehrere Wände eine oder mehrere Öffnungen aufweisen, durch die die eine oder mehreren Körperfluide in der Kammer durch die eine oder mehrere Wände in die Sekundärkammer fließen können.

4. Drainagebeutel nach einem der Ansprüche 1-3, wobei der mindestens eine Fluiddetektor an mindestens einer Innenfläche der einen oder mehreren Wände angeordnet ist und der mindestens eine Fluiddetektor an einem transparenten Abschnitt der einen oder mehreren Wände angeordnet ist.

5. Drainagebeutel nach einem der Ansprüche 1-4, wobei der mindestens eine Fluiddetektor Teil der einen oder der mehreren Wände bildet.

6. Drainagebeutel nach einem der Ansprüche 1-5, wobei der mindestens eine Fluiddetektor mindestens eine hydrochromatische Tinte einschließt.

7. Drainagebeutel nach einem der Ansprüche 1-6, wobei der mindestens eine Fluiddetektor mindestens einen pH-Indikator einschließt.

8. Drainagebeutel nach einem der Ansprüche 1-7, wobei der mindestens eine Fluiddetektor mindestens ein Hologramm einschließt.

9. Drainagebeutel nach einem der Ansprüche 1-8, wobei der mindestens eine Fluiddetektor einen Kondensator einschließt.

10. Drainagebeutel nach einem der Ansprüche 1-9, wobei der mindestens eine Fluiddetektor eine Menge der einen oder mehreren Körperfluide in der Kammer anzeigt.

11. Fluidsammelsystem (600; 700), umfassend:
einen Rollstuhl (644;744) einschließlich einer Vielzahl von Rädern (650), eines Sitzes (646;746) und einer Rückenlehne (648), wobei der Rollstuhl eine Vorderseite (652;752) und eine der Vorderseite gegenüberliegende Rückseite (654;754) bildet; und den Drainagebeutel (102-902) nach einem der Ansprüche 1-10.

12. Fluidsammelsystem (600; 700) nach Anspruch 11, das weiter Folgendes umfasst:
eine Fluidsammelanordnung; und
mindestens einen Drainageschlauch, der sich von dem Einlass des Drainagebeutels bis zu der Fluidsammelanordnung erstreckt.

13. Fluidsammelsystem nach Anspruch 12, wobei die Fluidsammelanordnung (962) Folgendes einschließt:
mindestens eine fluidundurchlässige Barriere (968), die mindestens eine Kammer, mindestens eine Öffnung und einen Fluidauslass definiert; und
mindestens ein poröses Material, das in der Kammer angeordnet ist.

14. Fluidsammelsystem (800; 900), umfassend:
eine Fluidsammelanordnung (862; 962);
den Drainagebeutel (102-902) nach einem der Ansprüche 1-10; und
mindestens einen Drainageschlauch (812; 912a), der sich von dem Einlass des Drainagebeutels bis zu der Fluidsammelanordnung erstreckt.

15. Verfahren zur Verwendung eines Drainagebeutels (102-902), wobei das Verfahren umfasst:
Aufnehmen einer Menge einer oder mehrerer Körperfluide in eine Kammer des Drainagebeutels, wobei der Drainagebeutel eine oder mehrere Wände (106-506; 906) einschließt, die die Kammer (109-509; 970) definieren, wobei die eine oder die mehreren Wände eine Vorderwand (114) und eine Rückwand (116) einschließen, wobei zumindest ein Abschnitt der Vorderwand (114) undurchsichtig ist; und
als Reaktion auf das Aufnehmen der Menge der Körperfluide in der Kammer, Anzeigen mindestens eines von einem Vorhandensein oder einer Menge von der einen oder den mehreren Körperfluide in der Kammer mit mindestens einem Fluiddetektor (104-704; 904),
wobei der mindestens eine Fluiddetektor undurchsichtig ist; und
wobei mindestens der Abschnitt der Vorderwand, der undurchsichtig ist, und der mindestens eine Fluiddetektor eine Sicht auf Körperfluide in der Kammer verhindern.

## Revendications

1. Poche de drainage (102-902), comprenant
une ou plusieurs parois (106-506 ; 906) incluant une paroi avant (114) et une paroi arrière (116), au moins une partie de la paroi avant est opaque, les une ou plusieurs parois définissant au moins :
une entrée (110 ; 910) ; et
une chambre (109-509, 970) ; et
au moins un détecteur de fluides (104-704,904) configuré pour détecter au moins l'une d'une présence ou d'une quantité d'un ou plusieurs fluides corporels dans la chambre, **caractérisée en ce que**
l'au moins un détecteur de fluides est opaque ; et
dans laquelle au moins la partie de la paroi avant qui est opaque et l'au moins un détecteur de fluides empêchent de voir les fluides corporels dans la chambre.

2. Poche de drainage selon la revendication 1, dans laquelle l'au moins un détecteur de fluides est disposé au moins partiellement sur au moins une surface extérieure (107-407) des une ou plusieurs parois.

3. Poche de drainage selon la revendication 2, dans laquelle l'au moins un détecteur de fluides et les une ou plusieurs parois définissent une chambre secondaire (426), les une ou plusieurs parois définissent un ou plusieurs trous qui permettent aux un ou plusieurs fluides corporels dans la chambre de s'écouler à travers les une ou plusieurs parois vers la chambre secondaire.

4. Poche de drainage selon l'une quelconque des revendications 1 à 3, dans laquelle l'au moins un détecteur de fluides est disposé sur au moins une surface intérieure des une ou plusieurs parois, et l'au moins un détecteur de fluides est disposé sur une partie transparente des une ou plusieurs parois.

5. Poche de drainage selon l'une quelconque des revendications 1 à 4, dans laquelle l'au moins un détecteur de fluides fait partie des une ou plusieurs parois.

6. Poche de drainage selon l'une quelconque des revendications 1 à 5, dans laquelle l'au moins un détecteur de fluides inclut au moins une encre hydrochromatique.

7. Poche de drainage selon l'une quelconque des revendications 1 à 6, dans laquelle l'au moins un détecteur de fluides inclut au moins un indicateur de pH.

8. Poche de drainage selon l'une quelconque des revendications 1 à 7, dans laquelle l'au moins un détecteur de fluides inclut au moins un hologramme.

9. Poche de drainage selon l'une quelconque des revendications 1 à 8, dans laquelle l'au moins un détecteur de fluides inclut un condensateur.

10. Poche de drainage selon l'une quelconque des revendications 1 à 9, dans laquelle l'au moins un détecteur de fluides indique une quantité des un ou plusieurs fluides corporels dans la chambre.

11. Système de collecte de fluides (600 ; 700), comprenant :
un fauteuil roulant (644 ; 744) incluant une pluralité de roues (650), un siège (646 ; 746) et un dossier (648), le fauteuil roulant formant un côté avant (652 ; 752) et un côté arrière (654 ; 754) opposé au côté avant ; et
la poche de drainage (102-902) selon l'une quelconque des revendications 1 à 10.

12. Système de collecte de fluides (600 ; 700) selon la revendication 11, comprenant en outre :
un ensemble de collecte de fluide ; et
au moins un tube de drainage s'étendant de l'entrée de la poche de drainage à l'ensemble de collecte de fluide.

13. Système de collecte de fluides selon la revendication 12, dans lequel l'ensemble de collecte de fluides (962) inclut :
au moins une barrière imperméable aux fluides (968) définissant au moins une chambre, au moins une ouverture et une sortie de fluide ; et
au moins un matériau poreux disposé dans la chambre.

14. Système de collecte de fluides (800 ; 900), comprenant :
un ensemble de collecte de fluides (862 ; 962) ;
la poche de drainage selon l'une quelconque des revendications 1 à 10 (102-902) ; et
au moins un tube de drainage (812 ; 912a) s'étendant de l'entrée de la poche de drainage à l'ensemble de collecte de fluide.

15. Procédé d'utilisation d'une poche de drainage (102-902), le procédé comprenant :
la réception d'une quantité d'un ou plusieurs fluides corporels dans une chambre de la poche de drainage, la poche de drainage incluant une ou plusieurs parois (106-506 ; 906) qui définissent la chambre (109-509 ; 970), les une ou plusieurs parois incluant une paroi avant (114) et une paroi arrière (116), au moins une partie de la paroi avant (114) est opaque ; et
en réponse à la réception de la quantité des fluides corporels dans la chambre, l'indication d'au moins l'une d'une présence ou d'une quantité des un ou plusieurs fluides corporels dans la chambre à l'aide d'au moins un détecteur de fluides (104-704 ; 904),
dans lequel l'au moins un détecteur de fluides est opaque ; et
dans lequel au moins la partie de la paroi avant qui est opaque et l'au moins un détecteur de fluides empêchent de voir les fluides corporels dans la chambre.
